# EUROPEAN PATENT SPECIFICATION

(11) **EP 4 282 354 B1**
(45) Date of publication and mention of the grant of the patent: **04.06.2025**
(21) Application number: 21921161.2
(22) Date of filing: 12.10.2021
(51) Int. Cl.: A61B 17/70, A61F 2/44, B29C 70/04, B29C 70/08

(54) **ROD FOR SPINAL FIXATION IMPLEMENT AND SPINAL FIXATION IMPLEMENT PROVIDED WITH SAME**
STANGE FÜR WIRBELSÄULENFIXATIONSINSTRUMENT UND WIRBELSÄULENFIXATIONSINSTRUMENT DAMIT
TIGE POUR DISPOSITIF DE FIXATION DE LA COLONNE VERTÉBRALE AINSI QUE DISPOSITIF DE FIXATION POUR LA COLONNE VERTÉBRALE COMPORTANT CELLE-CI

(30) Priority: 25.01.2021 JP 2021009692
(43) Date of publication of application: 29.11.2023
(73) Proprietor: Globeride, Inc., Higashikurume-shi, Tokyo 203-8511 (JP)
(72) Inventor: OIKAWA, Katsuhiro, Higashikurume-shi, Tokyo 203-8511 (JP); KAWAMURA, Takuji, Higashikurume-shi, Tokyo 203-8511 (JP)
(74) Representative: Boult Wade Tennant LLP
(86) International application number: PCT/JP2021/037721
(87) International publication number: WO 2022/158058

(56) References cited:
- WO-A1-2011/042998
- WO-A1-2014/162873
- DE-A1- 102013 013 024
- JP-A- H04 366 616
- JP-A- H11 296 033
- US-A1- 2011 060 365
- US-A1- 2011 106 162
- US-A1- 2011 152 937

## Description

### Technical Field

The present invention relates to a fixture rod used for a fixture configured to fix a spine and a spinal fixture comprising the same.

### Background Art

Conventionally, a fixture rod using metal as a fixture for fixing the spine has been known.

Further, as such a fixture rod, for example, Patent Literature 1 discloses a spinal pedicle rod comprising an internally reinforced polymer core at least partially encased in a polymer coating.

### Citation List

### Patent Literature

Patent Literature 1: Japanese Translation of PCT International Application Publication No. 2011-508623

### Summary of Invention

The present invention is defined in claim 1 while preferred embodiments are set forth in the dependent claims.

### Technical Problem

A fixture rod using metal is generally excellent in fixing force and strength, but has a problem that a magnetic field is affected by magnetization of the metal in the magnetic field at the time of imaging by MRI or the like, image disturbance occurs, and diagnosis based on a captured image is difficult. On the other hand, the rod disclosed in Patent Literature 1 does not have such a problem, but has a problem that it is difficult to reliably achieve uniform bonding even if an adhesive is used for bonding between a polymer core material and a covering layer thereof, and it is difficult to obtain stable bonding strength.

An object of the present invention is to provide a fixture rod that is excellent in bonding strength between a core material and a reinforcing fiber layer and has high rigidity and high durability against a deformation load, and a spinal fixture comprising the same. Purposes of the present invention other than this object will be clarified by referring to the overall description disclosed herein.

### Solution to Problem

A fixture rod according to one embodiment of the present invention comprises: a core member containing a resin; and a reinforcing fiber layer provided on the core member, and is configured such that the resin of the core member and a resin of the reinforcing fiber layer are the same resin, or the resin of the core member and the resin of the reinforcing fiber layer are different resins, and a critical surface tension of each of the resin of the core member and the resin of the reinforcing fiber layer is 20 mN/m or more.

In the fixture rod according to one embodiment of the present invention, one recess or a plurality of recesses are formed on an outer surface of the core member.

In the fixture rod according to one embodiment of the present invention, the recess is formed in a circumferential direction of the core member.

In the fixture rod according to one embodiment of the present invention, the recess is formed in an axial direction of the core member.

In the fixture rod according to one embodiment of the present invention, the recess is formed in a direction inclined with respect to a circumferential direction of the core member.

In the fixture rod according to one embodiment of the present invention, the recesses comprise two or more recesses formed in different directions.

In the fixture rod according to one embodiment of the present invention, a depth of the recess is in a range of 3 µm to 200 µm.

In the fixture rod according to one embodiment of the present invention, the core member is formed using the resin containing a fiber.

In the fixture rod according to one embodiment of the present invention, the fiber of the core member is partially exposed from the core member.

In the fixture rod according to one embodiment of the present invention, the fiber of the core member is a long fiber. Further, in the fixture rod according to one embodiment of the present invention, the fiber of the core member is a short fiber.

The fixture rod according to one embodiment of the present invention is configured such that the resin of the core member is any of epoxy, phenol, unsaturated polyester, PA, PC, PPSU, POM, PP, PE, ABS, PS, PAEK, or PEEK.

A spinal fixture according to one embodiment of the present invention comprises any of the fixture rods described above.

### Advantageous Effects of Invention

According to each of the above embodiments of the present invention, it is possible to provide the fixture rod that is excellent in bonding strength between the core material and the reinforcing fiber layer and has high rigidity and high durability against the deformation load, and the spinal fixture comprising the same.

### Brief Description of Drawings

Fig. 1 is a view illustrating a spinal fixture 10 comprising a fixture rod according to one embodiment of the present invention.
Fig. 2 is a view schematically illustrating a cross section of the fixture rod according to one embodiment of the present invention taken along a plane perpendicular to a central axis thereof.
Fig. 3 is a view for describing a core member of the fixture rod according to one embodiment of the present invention.
Fig. 4 is a view for describing a core member of the fixture rod according to one embodiment of the present invention.
Fig. 5 is a view for describing a core member of the fixture rod according to one embodiment of the present invention.
Fig. 6 is a view for describing a core member of the fixture rod according to one embodiment of the present invention.
Fig. 7a is a view for describing a core member of the fixture rod according to one embodiment of the present invention.
Fig. 7b is a view for describing a core member of the fixture rod according to one embodiment of the present invention.
Fig. 8a is a view for describing a core member of the fixture rod according to one embodiment of the present invention.
Fig. 8b is a view for describing a core member of the fixture rod according to one embodiment of the present invention.
Fig. 9a is a view for describing a method for molding the fixture rod according to one embodiment of the present disclosure (method not claimed).
Fig. 9b is a view for describing a method for molding the fixture rod according to one embodiment of the present disclosure (method not claimed).
Fig. 9c is a view for describing a method for molding the fixture rod according to one embodiment of the present disclosure (method not claimed).
Fig. 9d is a view for describing a method for molding the fixture rod according to one embodiment of the present disclosure (method not claimed).
Fig. 9e is a view for describing a method for molding the fixture rod according to one embodiment of the present disclosure (method not claimed).
Fig. 9f is a view for describing a method for molding the fixture rod according to one embodiment of the present disclosure (method not claimed).
Fig. 9g is a view for describing a method for molding the fixture rod according to one embodiment of the present disclosure (method not claimed).

### Description of Embodiments

Hereinafter, an embodiment of a fixture rod according to the present invention will be specifically described with reference to the accompanying drawings. Components common in a plurality of drawings are assigned with the same reference signs throughout the plurality of drawings. It should be noted that each of the drawings is not always illustrated in a precise aspect ratio for the convenience of description.

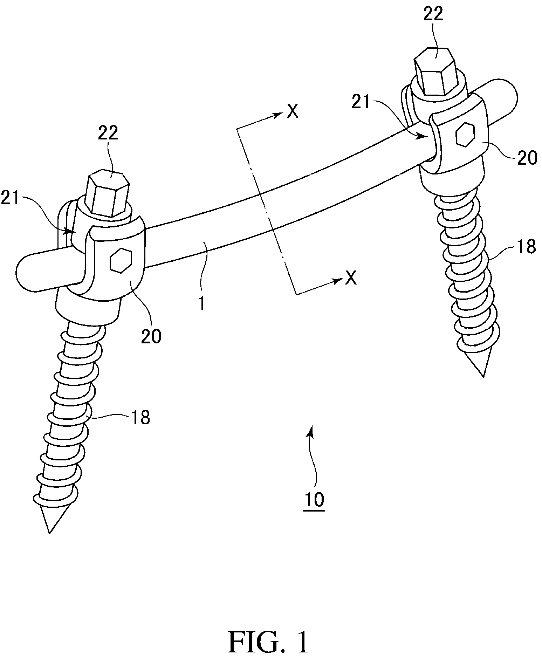

Fig. 1 is a view illustrating a spinal fixture 10 comprising a fixture rod 1 according to one embodiment of the present invention. As illustrated in the drawing, the spinal fixture 10 comprises a plurality of screw members 18 (two screw members 18 in the example illustrated in the drawing) to be fixed to the bone of the spine, a plurality of rod fixing members 20 (two rod fixing members 20 in the example illustrated in the drawing) attached to the screw members 18 and each comprising a recess 21 for receiving the fixture rod and a pressing member 22, and the fixture rod 1 inserted into the recess 21 of the plurality of rod fixing members 20 and fixed by the pressing member 22.

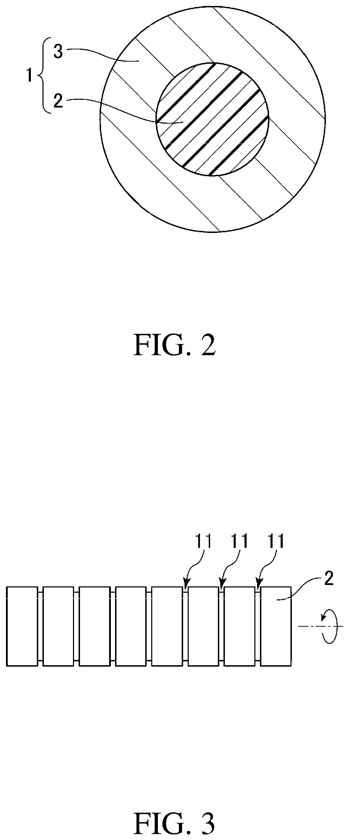

Next, the fixture rod 1 according to one embodiment of the present invention used for the spinal fixture 10 will be described with reference to Fig. 2.

Fig. 2 illustrates the fixture rod 1 illustrated in Fig. 1 as viewed in X-X section illustrated in the same drawing.

As illustrated in the drawing, the fixture rod 1 according to one embodiment of the present invention comprises: a core member 2 containing a resin; and a reinforcing fiber layer 3 provided on the core member 2, and is configured such that the resin of the core member 2 and a resin of the reinforcing fiber layer 3 are the same resin, or the resin of the core member and the resin of the reinforcing fiber layer are different resins, and a critical surface tension of each of the resin of the core member and the resin of the reinforcing fiber layer is 20 mN/m or more.

According to the fixture rod 1 according to one embodiment of the present invention, it is possible to provide the fixture rod that is excellent in bonding strength between a core material and the reinforcing fiber layer and has high rigidity and high durability against a deformation load. More specifically, the affinity between the core material and the reinforcing fiber layer is improved, and the bonding strength between the core material and the reinforcing fiber layer is excellent when the resin of the core member and the resin of the reinforcing fiber layer each having the critical surface tension of 20 mN/m or more are adopted whether the same resin or different resins are used, Further, a solid double structure is adopted, and a material having a large average bending elastic modulus is used for an outer layer as will be described later, and thus, it is possible to provide the fixture rod having excellent bending rigidity and crushing strength of the entire rod. Here, the average bending elastic modulus refers to a value calculated by dividing the bending rigidity of the entire corresponding portion by a second moment of the corresponding portion.

Here, even if the resin of the core member and the resin of the reinforcing fiber layer are different resins, it has been confirmed that the critical surface tension of the resin exceeds desired bonding performance in bonding between different types of materials when the critical surface tensions of the resin of the core member and the resin of the reinforcing fiber layer are 20 mN/m or more, and it has been found that a special process, such as a chemical solution treatment or a plasma treatment, for bonding is unnecessary. More specifically, for example, critical surface tensions of polypropylene (PP), polyethylene (PE), polystyrene (PS), polyoxymethylene (POM), polyethylene terephthalate (PET), and nylon 66 are 22 to 29 mN/m, 31 mN/m, 33 mN/m, 36 to 38 mN/m, 43 mN/m, and 46 mN/m, respectively, and it has been found that favorable bonding performance is exhibited due to the critical surface tensions of the resins even if the resin of the core member and the resin of the reinforcing fiber layer are different resins. On the other hand, a critical surface tension of polytetrafluoroethylene paraffin (PTFE) is 18.5 mN/m, and it has been found that a special process, such as a chemical liquid treatment or a plasma treatment, for bonding is required because the critical surface tension of the resin is lower than the desired bonding performance in bonding between different types of materials. However, this is not applied when the resin of the core member and the resin of the reinforcing fiber layer are the same resin.

In the fixture rod 1 according to one embodiment of the present invention, a thermosetting resin (for example, epoxy, phenol, unsaturated polyester, or the like) or a thermoplastic resin (for example, PA, PC, PPSU, POM, PP, PE, ABS, PS, PAEK, PEEK, or the like) is used as the resin of the core member 2.

In the fixture rod 1 according to one embodiment of the present invention, the core member 2 can be formed using a resin containing fibers. In such a case, it is configured such that the fiber is any of carbon, glass, aramid, boron, or SiC, and the resin is a thermosetting resin (for example, epoxy, phenol, unsaturated polyester, or the like) or a thermoplastic resin (for example, PA, PC, PPSU, POM, PP, PE, ABS, PS, PAEK, PEEK, or the like). With this configuration, it is possible to increase the bending rigidity and the strength of the core member.

In the fixture rod 1 according to one embodiment of the present invention, the reinforcing fiber layer 3 is a fiber-reinforced resin, carbon, glass, boron, SiC, or aramid is used as a fiber, and a thermosetting resin (for example, epoxy, phenol, unsaturated polyester, or the like) or a thermoplastic resin (for example, PA, PC, PPSU, POM, PP, PE, ABS, PS, PAEK, PEEK, or the like) is used as a resin. With this configuration, it is possible to increase the bending rigidity and the strength of the reinforcing fiber layer.

It is configured such that the fixture rod 1 according to one embodiment of the present invention comprises a covering layer provided on the reinforcing fiber layer 3. The covering layer can be formed using, for example, epoxy, phenol, unsaturated polyester, PA, PC, PPSU, POM, PP, PE, ABS, PS, PAEK, or PEEK, but is not limited thereto.

Next, the core member 2 of the fixture rod 1 according to one embodiment of the present invention used for the spinal fixture 10 will be described with reference to Figs. 3 to 8. In the fixture rod 1 according to one embodiment of the present invention, one or a plurality of recesses are formed on an outer surface of the core member 2. As a result, the contact surface area between the core member 2 and the reinforcing fiber layer 3 increases during molding, so that the bonding strength between the core material and the reinforcing fiber layer can be significantly improved. This will be described more specifically below.

As illustrated in Fig. 3, recesses (circumferential recesses) 11 are formed in a circumferential direction of the core member 2 in the fixture rod 1 according to one embodiment of the present invention. Although seven recesses 11 are formed in the example illustrated in the drawing, any desired number of recesses can be provided, and the number is not limited to a specific number. Further, the recess 11 can be formed in the whole or a part of the core member 2 in the circumferential direction Alternatively, the recess 11 may be intermittently formed in the whole or a part of the core member 2 in the circumferential direction. When the recess is provided in the circumferential direction of the core member in this manner, the bonding area increases, and axial displacement between the core member 2 and the reinforcing fiber layer 3 can be suppressed.

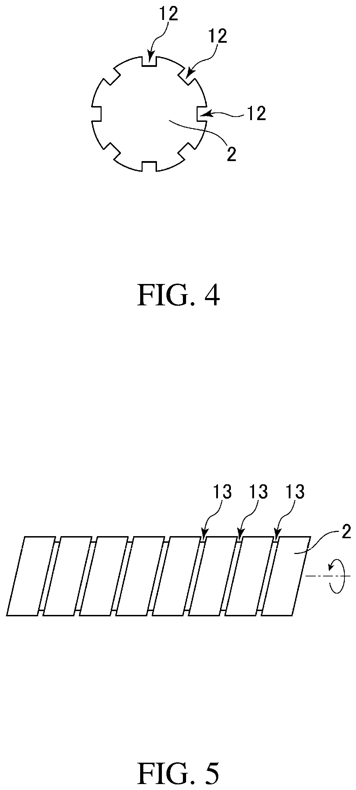

Next, as illustrated in Fig. 4, recesses (axial recesses) 12 are formed in an axial direction of the core member 2 in the fixture rod 1 according to one embodiment of the present invention. Although eight recesses 12 are formed in the example illustrated in the drawing, any desired number of recesses can be provided, and the number is not limited to a specific number. Further, the recess 12 can be formed in the whole or a part of the core member 2 in the axial direction Alternatively, the recess 12 may be intermittently formed in the whole or a part of the core member 2 in the axial direction. When the recess is provided in the axial direction of the core member in this manner, the bonding area increases, and rotational displacement between the core member 2 and the reinforcing fiber layer 3 can be suppressed.

Next, as illustrated in Fig. 5, recesses (inclined-direction recesses) 13 are formed so as to be inclined with respect to the circumferential direction of the core member 2 in the fixture rod 1 according to one embodiment of the present invention. Although seven recesses 13 are formed in the example illustrated in the drawing, any desired number of recesses can be provided, and the number is not limited to a specific number. Further, the recess 13 can be formed on the whole or a part of the circumference of the core member 2. Alternatively, the recess 13 may be intermittently formed on the whole or a part of the circumference of the core member 2. When the recess is provided in a direction inclined with respect to the circumferential direction of the core member in this manner, the bonding area increases, and the axial displacement and rotational displacement between the core member 2 and the reinforcing fiber layer 3 can be suppressed.

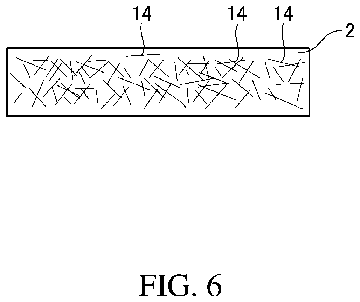

Next, as illustrated in Fig. 6, recesses (different-direction recesses) 14 are formed on the surface of the core member 2 so as to comprise two or more recesses formed in different directions in the fixture rod 1 according to one embodiment of the present invention. Although many recesses 14 are formed in the example illustrated in the drawing, any desired number of recesses can be provided, and the number is not limited to a specific number. Further, in a case where there are three or more recesses 14, two or more recesses 14 thereof may be formed in the same direction. Further, the recesses 14 can be formed on the whole or a part of the surface of the core member 2. Alternatively, the recess 11 may be intermittently formed on the whole or a part of the surface of the core member 2. When the recesses are provided in different directions in this manner, it is possible to suppress displacement in a plurality of different directions.

In the fixture rod according to one embodiment of the present invention, a depth of the recess is in a range of 3 µm to 200 µm. As a result, it is possible to set an appropriate range in which the displacement between the core member 2 and the reinforcing fiber layer 3 is suppressed while suppressing a change and a variation in the rigidity due to the recess.

In the fixture rod 1 according to one embodiment of the present invention, the core member 2 can be formed using a resin containing fibers, and is configured such that the fibers of the core member are short fibers. When the short fibers are used, fiber directions can be randomly oriented, and reinforcement in all directions is possible.

In the fixture rod 1 according to one embodiment of the present invention, the core member 2 can be formed using a resin containing fibers, and is configured such that the fibers of the core member are long fibers. As a result, the bending rigidity can be effectively improved.

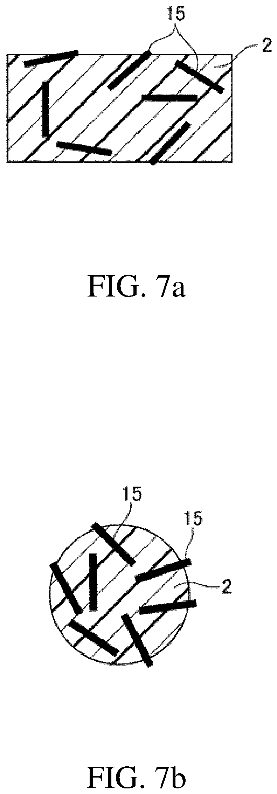

Next, as described above, the core member 2 can be formed using a resin containing fibers in the fixture rod 1 according to one embodiment of the present invention. In such a case, as illustrated in Fig. 7, fibers (short fibers) 15 of the core member 2 are partially exposed from the surface of the core member in the fixture rod 1 according to one embodiment of the present invention. When the short fibers 15 are exposed in this manner, minute irregularities are generated on the surface of the core member 2, so that the displacement between the core member 2 and the reinforcing fiber layer 3 can be suppressed.

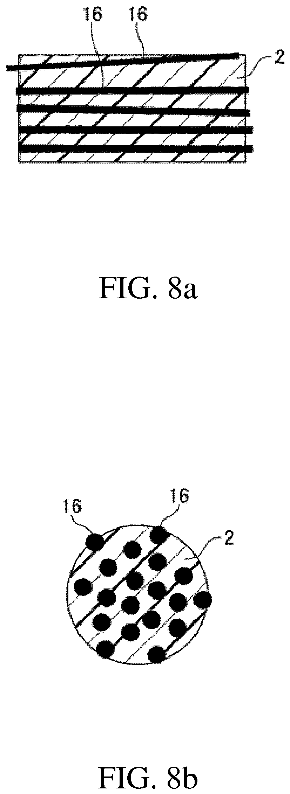

Next, as described above, the core member 2 can be formed using a resin containing fibers in the fixture rod 1 according to one embodiment of the present invention. In such a case, as illustrated in Fig. 8, fibers (long fibers) 16 of the core member 2 are partially exposed from the surface of the core member 2 in the fixture rod 1 according to one embodiment of the present invention. When the long fibers 16 are exposed in this manner, minute irregularities are generated on the surface of the core member 2, so that the displacement between the core member 2 and the reinforcing fiber layer 3 can be suppressed.

The fixture rod 1 according to one embodiment of the present invention is configured such that fibers of the reinforcing fiber layer 3 are long fibers. Since the fibers of the reinforcing fiber layer 3 are long fibers, it is possible to further increase the bending rigidity and the strength.

Further, the fixture rod 1 according to one embodiment of the present invention is configured such that a fiber content of one or more layers included in the reinforcing fiber layer 3 is 60% by weight or more. It is possible to form the fixture rod 1 having high rigidity and excellent durability with the fiber layer in which the long fibers are filled at high density in this manner.

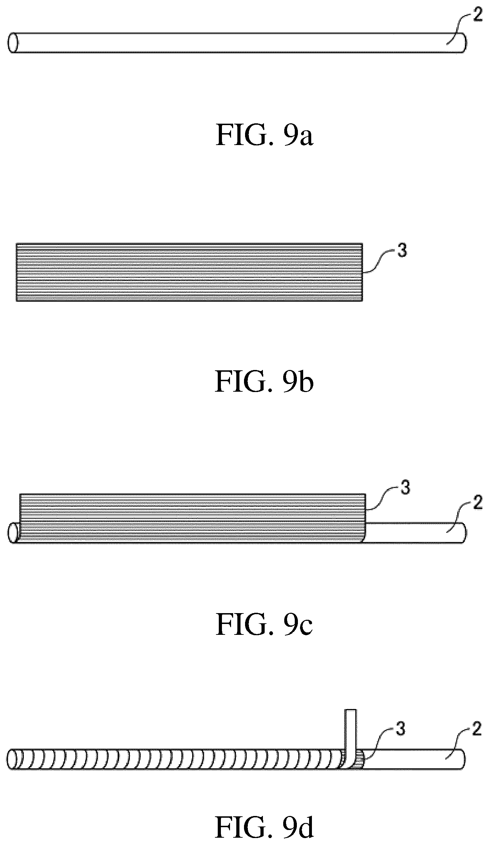

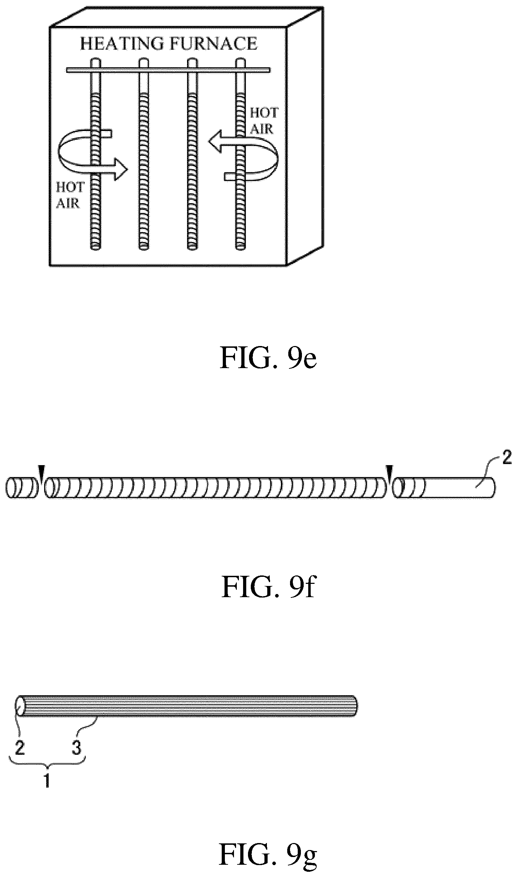

Next, a method for manufacturing the fixture rod 1 according to one embodiment of the present disclosure (method not claimed) will be described with reference to Fig. 9. First, as Step 1, a core member (core material) (including each of modes in Figs. 3 to 8 described above) is prepared (Fig. 9a). Next, as Step 2, a fiber-reinforced resin material is prepared (Fig. 9b). Next, as Step 3, the fiber-reinforced resin material is wound around the core material to form a fiber-reinforced resin material integrated member (Fig. 9c).

Next, in Step 4, a tape is wound around an outer surface of a fiber-reinforced resin material integrated member as an outer die (Fig. 9d). Next, in Step 5, the fiber-reinforced resin material integrated member around which the tape is wound is fired (molded) (Fig. 9e). Thereafter, as Step 6, the fiber-reinforced resin material integrated member after firing is taken out, and an unnecessary portion is cut (Fig. 9f). Finally, as Step 7, the tape of the fiber-reinforced resin material integrated member from which the unnecessary portion has been cut is removed, whereby the fixture rod 1 according to one embodiment of the present disclosure (method not claimed) comprising the core member and a fiber-reinforced resin layer can be obtained (Fig. 9g).

With the fixture rod 1 according to one embodiment of the present disclosure (method not claimed) formed in this manner, it is possible to provide the fixture rod that is excellent in bonding strength between the core material and a reinforcing fiber layer and has high rigidity and high durability against a deformation load. More specifically, the affinity between the core material and the reinforcing fiber layer is improved, and the bonding strength between the core material and the reinforcing fiber layer is excellent when the resin of the core member and the resin of the reinforcing fiber layer each having the critical surface tension of 20 mN/m or more are adopted whether the same resin or different resins are used. Further, a solid double structure is adopted, and a material having a large average bending elastic modulus is used for an outer layer as will be described later, and thus, it is possible to provide the fixture rod having excellent bending rigidity and crushing strength of the entire rod. Here, the average bending elastic modulus refers to a value calculated by dividing the bending rigidity of the entire corresponding portion by a second moment of the corresponding portion.

The spinal fixture 10 according to one embodiment of the present invention comprises any of the fixture rods 1 described above.

Dimensions, materials, and arrangements of the components described in this specification are not limited to those explicitly described in the embodiments, and the components may be modified to have any dimensions, materials, and arrangements that may fall within the scope of the present invention. Further, components not explicitly described herein can be added to the described embodiments, or some of the components described in each embodiment can be omitted.

### Reference Signs List

- 1: Fixture rod
- 2: Core member
- 3: Reinforcing fiber layer
- 10: Spinal fixture
- 11: Recess (circumferential recess)
- 12: Recess (axial recess)
- 13: Recess (inclined-direction recess)
- 14: Recesses (different-direction recesses)
- 15: Fiber (short fiber)
- 16: Fiber (long fiber)
- 18: Screw member
- 20: Rod fixing member
- 21: Recess
- 22: Pressing member

## Claims

1. A fixture rod (1) comprising:
a core member (2) containing a resin; and a reinforcing fiber layer (3) provided on the core member (2),
wherein the resin of the core member (2) and a resin of the reinforcing fiber layer (3) are an identical resin, or the resin of the core member (2) and the resin of the reinforcing fiber layer (3) are different resins, and **characterized in that**:
a critical surface tension of each of the resin of the core member (2) and the resin of the reinforcing fiber (3) layer is 20 mN/m or more; and
the core member (2) is formed using a resin containing fibers (15, 16), the fibers (15, 16) of the core member (2) comprising fibers partially exposed from the core member (2), the partially exposed fibers being long fibers (16) or short fibers (15).

2. The fixture rod (1) according to claim 1, wherein one recess or a plurality of recesses (11, 12, 13, 14) are formed on an outer surface of the core member (2).

3. The fixture rod (1) according to claim 2, wherein the recess (11) is formed in a circumferential direction of the core member (2).

4. The fixture rod (1) according to claim 2, wherein the recess (12) is formed in an axial direction of the core member (2).

5. The fixture rod (1) according to claim 2, wherein the recess (13) is formed in a direction inclined with respect to a circumferential direction of the core member (2).

6. The fixture rod (1) according to claim 2, wherein the recesses (14) comprise two or more recesses (14) formed in different directions.

7. The fixture rod (1) according to any one of claims 2 to 5, wherein a depth of the recess (11, 12, 13, 14) is in a range of 3 µm to 200 µm.

8. The fixture rod (1) according to any one of claims 1 to 7, wherein the fibers of the core member (2) are long fibers (16).

9. The fixture rod (1) according to any one of claims 1 to 7, wherein the fibers of the core member (2) are short fibers (15).

10. The fixture rod (1) according to any one of claims 1 to 9, wherein the resin of the core member (2) is any of epoxy, phenol, unsaturated polyester, PA, PC, PPSU, POM, PP, PE, ABS, PS, PAEK, or PEEK.

11. A spinal fixture (10) comprising the fixture rod (1) according to any one of claims 1 to 10.

## Patentansprüche

1. Fixationsstange (1), umfassend:
ein Kernelement (2), das ein Harz enthält; und eine Verstärkungsfaserschicht (3), die auf dem Kernelement (2) vorgesehen ist,
wobei das Harz des Kernelements (2) und ein Harz der Verstärkungsfaserschicht (3) ein identisches Harz sind, oder das Harz des Kernelements (2) und das Harz der Verstärkungsfaserschicht (3) unterschiedliche Harze sind, und **dadurch gekennzeichnet, dass**:
eine kritische Oberflächenspannung sowohl des Harzes des Kernelements (2) als auch des Harzes der Verstärkungsfaserschicht (3) 20 mN/m oder mehr beträgt, und
das Kernelement (2) unter Verwendung eines Harzes gebildet ist, das Fasern (15, 16) enthält, wobei die Fasern (15, 16) des Kernelements (2) Fasern umfassen, die teilweise von dem Kernelement (2) freigelegt sind, wobei die teilweise freigelegten Fasern lange Fasern (16) oder kurze Fasern (15) sind.

2. Fixationsstange (1) nach Anspruch 1, wobei eine Aussparung oder eine Mehrzahl von Aussparungen (11, 12, 13, 14) an einer Außenfläche des Kernelements (2) ausgebildet sind.

3. Fixationsstange (1) nach Anspruch 2, wobei die Aussparung (11) in einer Umfangsrichtung des Kernelements (2) ausgebildet ist.

4. Fixationsstange (1) nach Anspruch 2, wobei die Aussparung (12) in einer axialen Richtung des Kernelements (2) ausgebildet ist.

5. Fixationsstange (1) nach Anspruch 2, wobei die Aussparung (13) in einer Richtung ausgebildet ist, die in Bezug auf eine Umfangsrichtung des Kernelements (2) geneigt ist.

6. Fixationsstange (1) nach Anspruch 2, wobei die Aussparungen (14) zwei oder mehr Aussparungen (14) umfassen, die in unterschiedlichen Richtungen ausgebildet sind.

7. Fixationsstange (1) nach einem der Ansprüche 2 bis 5, wobei eine Tiefe der Aussparung (11, 12, 13, 14) in einem Bereich von 3 µm bis 200 µm liegt.

8. Fixationsstange (1) nach einem der Ansprüche 1 bis 7, wobei die Fasern des Kernelements (2) lange Fasern (16) sind.

9. Fixationsstange nach einem der Ansprüche 1 bis 7, wobei die Fasern des Kernelements (2) kurze Fasern (15) sind.

10. Fixationsstange nach einem der Ansprüche 1 bis 9, wobei das Harz des Kernelements (2) eines der folgenden ist: Epoxid, Phenol, ungesättigter Polyester, PA, PC, PPSU, POM, PP, PE, ABS, PS, PAEK oder PEEK.

11. Wirbelsäulenfixation (10), welche die Fixationsstange (1) nach einem der Ansprüche 1 bis 10 umfasst.

## Revendications

1. Tige de dispositif de fixation (1) comprenant :
un élément central (2) contenant une résine ; et une couche de fibres de renfort (3) prévue sur l'élément central (2),
dans laquelle la résine de l'élément central (2) et une résine de la couche de fibres de renfort (3) sont une résine identique, ou la résine de l'élément central (2) et la résine de la couche de fibres de renfort (3) sont des résines différentes, et **caractérisée en ce que** :
une tension superficielle critique de chacune parmi la résine de l'élément central (2) et la résine de la couche de fibres de renfort (3) est de 20 mN/m ou plus ; et
l'élément central (2) est formé à l'aide d'une résine contenant des fibres (15, 16), les fibres (15, 16) de l'élément central (2) comprenant des fibres partiellement exposées à partir de l'élément central (2), les fibres partiellement exposées étant des fibres longues (16) ou des fibres courtes (15).

2. Tige de dispositif de fixation (1) selon la revendication 1, dans laquelle un évidement ou une pluralité d'évidements (11, 12, 13, 14) sont formés sur une surface externe de l'élément central (2).

3. Tige de dispositif de fixation (1) selon la revendication 2, dans laquelle l'évidement (11) est formé dans une direction circonférentielle de l'élément central (2).

4. Tige de dispositif de fixation (1) selon la revendication 2, dans laquelle l'évidement (12) est formé dans une direction axiale de l'élément central (2).

5. Tige de dispositif de fixation (1) selon la revendication 2, dans laquelle l'évidement (13) est formé dans une direction inclinée par rapport à une direction circonférentielle de l'élément central (2).

6. Tige de dispositif de fixation (1) selon la revendication 2, dans laquelle les évidements (14) comprennent deux évidements (14) ou plus formés dans des directions différentes.

7. Tige de dispositif de fixation (1) selon l'une quelconque des revendications 2 à 5, dans laquelle une profondeur de l'évidement (11, 12, 13, 14) est dans une plage de 3 µm à 200 µm.

8. Tige de dispositif de fixation (1) selon l'une quelconque des revendications 1 à 7, dans laquelle les fibres de l'élément central (2) sont des fibres longues (16).

9. Tige de dispositif de fixation (1) selon l'une quelconque des revendications 1 à 7, dans laquelle les fibres de l'élément central (2) sont des fibres courtes (15).

10. Tige de dispositif de fixation (1) selon l'une quelconque des revendications 1 à 9, dans laquelle la résine de l'élément central (2) est l'une quelconque parmi époxy, phénol, polyester insaturé, PA, PC, PPSU, POM, PP, PE, ABS, PS, PAEK, ou PEEK.

11. Dispositif de fixation de colonne vertébrale (10) comprenant la tige de dispositif de fixation (1) selon l'une quelconque des revendications 1 à 10.
